# EUROPEAN PATENT APPLICATION

(11) **EP 3 547 176 A1**
(43) Date of publication of application: **02.10.2019**
(21) Application number: 17874417.3
(22) Date of filing: 16.11.2017
(51) Int. Cl.: G06F 19/00, G06Q 50/22, A61B 5/021, A61B 5/026, A61B 5/024

(54) **APPARATUS AND METHOD FOR DETERMINING CIRCULATORY DISEASE POTENTIAL**

(30) Priority: 25.11.2016 KR 20160158494
(71) Applicant: Korea Institute of Science and Technology, Seoul 02792 (KR)
(72) Inventor: YOON, Chang No, Seoul 02792 (KR); SEO, Hong Seog, Seoul 04322 (KR); HAN, Won Seok, Gwangmyeong-si Gyeonggi-do 14287 (KR); LEE, Jin Kak, Hwaseong-si Gyeonggi-do 18297 (KR); LEE, Jun Seok, Seoul 02792 (KR)
(74) Representative: Walaski, Jan Filip
(86) International application number: PCT/KR2017/013026
(87) International publication number: WO 2018/097541

(57) **Abstract**

Provided is a method of determining circulatory disease potential. The method includes: a user data receiving operation of receiving user data about a blood pressure and a blood flow of a user; a pattern calculating operation of calculating a three-dimensional (3D) user pattern for the user based on the received user data; a pattern comparing operation of comparing a comparison target pattern calculated based on data about blood pressures and blood flows of a normal person and a circulatory patient stored in a database, with the calculated user pattern; and a result output operation of outputting, based on a result of the comparison, circulatory disease potential visualizing to which of the normal person and the circulatory patient stored in the database the user is closer.

## Description

### TECHNICAL FIELD

The present disclosure relates to an apparatus and method for determining circulatory disease potential, and a computer-readable recording medium having recorded thereon a program for performing the method.

### BACKGROUND ART

As people's interest in health increases day by day, more and more apparatuses are able to easily measure health-related indexes. For example, as various measurement apparatuses such as blood pressure meters, pulse meters, and blood oxygen saturation meters are gradually advanced and more lightweight, people not only may use measurement apparatuses by visiting certain places but also may use measurement apparatuses while easily carrying them.

However, until now, in view of two aspects of portability and technical limit, portable health index measurement apparatuses may merely display a result thereof immediately when a user applies an input thereto according to a certain usage method, and rare portable apparatuses may objectively inform a comparison result in combination with actual data and their prices are considerably high.

Meanwhile, as multifunctional intelligent complex terminals such as smart phones having various wired/wireless communication functions are widely popularized, various apparatuses capable of being added and used in smart phones are also increasingly used. In this regard, there is a need for an apparatus that may provide accurate and useful information to a user by using all of an accurate measurement function of a portable health index meter and an advanced operation processing function and a display function that are included in a smart phone.

### DESCRIPTION OF EMBODIMENTS

### TECHNICAL PROBLEM

Provided is an apparatus and method for determining circulatory disease potential, which may determine how easily persons having a similar constitution to the user catch a circulatory disease based on the blood pressure and pulse values of the user obtained through a portable measurement apparatus.

### SOLUTION TO PROBLEM

According to an aspect of the present disclosure, a method of determining circulatory disease potential includes: a user data receiving operation of receiving user data about a blood pressure and a blood flow of a user; a pattern calculating operation of calculating a three-dimensional (3D) user pattern for the user based on the received user data; a pattern comparing operation of comparing a comparison target pattern calculated based on data about blood pressures and blood flows of a normal person and a circulatory patient stored in a database, with the calculated user pattern; and a result output operation of outputting, based on a result of the comparison, circulatory disease potential visualizing to which of the normal person and the circulatory patient stored in the database the user is closer.

According to another aspect of the present disclosure, an apparatus for determining circulatory disease potential includes: a database storing a comparison target pattern calculated based on data about blood pressures and blood flows of a normal person and a circulatory patient; a user data receiver receiving user data about a blood pressure and a blood flow of a user; a pattern calculator calculating a three-dimensional (3D) user pattern for the user based on the received user data; a pattern comparator comparing the comparison target pattern stored in the database, with the calculated user pattern; and a result outputter outputting, based on a result of the comparison, circulatory disease potential visualizing to which of the normal person and the circulatory patient stored in the database the user is closer.

### ADVANTAGEOUS EFFECTS OF DISCLOSURE

According to the present disclosure, it may be possible to visually identify a similarity map representing the difference between the constitutions of the user and all the persons stored in the database and to intuitively detect the potential (potential risk) of a circulatory disease of the user.

Also, by inputting information about the number of groups, the user may specify a group including only some persons determined to be similar to the constitution of the user among the persons stored in the database and identify only information of the persons belonging to the group, thus making it possible to quickly cope with a circulatory disease that the user may catch.

### BRIEF DESCRIPTION OF DRAWINGS

FIG. 1 is a block diagram illustrating an example of a circulatory disease potential determining apparatus included in a user terminal.
FIG. 2 is a block diagram illustrating another example of a circulatory disease potential determining apparatus according to the present disclosure.
FIG. 3 is a diagram illustrating an example of a three-dimensional (3D) user pattern calculated by a pattern calculator.
FIG. 4 is a diagram illustrating an example of a similarity map output by a result outputter.
FIG. 5 is a diagram illustrating an example of a similarity map output when a user data receiver receives information about the number of groups.
FIG. 6 is a diagram illustrating in detail a first group to which the user belongs.
FIG. 7 is a flowchart illustrating an example of a circulatory disease potential determining method according to the present disclosure.

### BEST MODE

According to an aspect of the present disclosure, a method of determining circulatory disease potential includes: a user data receiving operation of receiving user data about a blood pressure and a blood flow of a user; a pattern calculating operation of calculating a three-dimensional (3D) user pattern for the user based on the received user data; a pattern comparing operation of comparing a comparison target pattern calculated based on data about blood pressures and blood flows of a normal person and a circulatory patient stored in a database, with the calculated user pattern; and a result output operation of outputting, based on a result of the comparison, circulatory disease potential visualizing to which of the normal person and the circulatory patient stored in the database the user is closer.

The received user data may be data measured periodically at least two times for an input time.

The pattern calculating operation may include: a maximum blood pressure pattern calculating operation of calculating a user maximum blood pressure pattern based on user data about the maximum blood pressure of the user; a minimum blood pressure pattern calculating operation of calculating a user minimum blood pressure pattern based on user data about the minimum blood pressure of the user; a heartbeat pattern calculating operation of calculating a heartbeat pattern based on user data about the heartbeat of the user; and an individual pattern arranging operation of arranging the calculated user maximum blood pressure pattern, user minimum blood pressure pattern, and heartbeat pattern together in a single 3D space.

The pattern comparing operation may include: a 3D spatial pattern comparing operation of comparing the comparison target pattern with the calculated user pattern based on a position in a 3D space; and a relative position calculating operation of calculating a distance and direction of the calculated user pattern and the comparison target pattern based on a result of the comparison in the 3D spatial pattern comparing operation, and the result output operation may include: a relative position determining operation of determining a relative position of the normal person and the circulatory patient with respect to the user according to the calculated distance and direction; and an arrangement result output operation of arranging the user, the normal person, and the circulatory patient according to the determined relative position and outputting a result of the arrangement.

The method may further include a group number information receiving operation of receiving information about a group number, wherein the result output operation may detect a normal person and a circulatory patient in a group to which the user belongs, among at least two groups classified according to the received group number and the result of the comparison and output the detected normal person and circulatory patient together with the user.

According to another aspect of the present disclosure, an apparatus for determining circulatory disease potential includes: a database storing a comparison target pattern calculated based on data about blood pressures and blood flows of a normal person and a circulatory patient; a user data receiver receiving user data about a blood pressure and a blood flow of a user; a pattern calculator calculating a three-dimensional (3D) user pattern for the user based on the received user data; a pattern comparator comparing the comparison target pattern stored in the database, with the calculated user pattern; and a result outputter outputting, based on a result of the comparison, circulatory disease potential visualizing to which of the normal person and the circulatory patient stored in the database the user is closer.

The received user data may be data measured periodically at least two times for an input time.

The pattern calculator may include: a maximum blood pressure pattern calculator calculating a user maximum blood pressure pattern based on user data about the maximum blood pressure of the user; a minimum blood pressure pattern calculator calculating a user minimum blood pressure pattern based on user data about the minimum blood pressure of the user; a heartbeat pattern calculator calculating a heartbeat pattern based on user data about the heartbeat of the user; and an individual pattern arranger arranging the calculated user maximum blood pressure pattern, user minimum blood pressure pattern, and user heartbeat pattern together in a single 3D space.

The pattern comparator may include: a 3D spatial pattern comparator comparing the comparison target pattern with the calculated user pattern based on a position in a 3D space; and a relative position calculator calculating a distance and direction of the calculated user pattern and the comparison target pattern based on a result of the comparison by the 3D spatial pattern comparator, and the result outputter may include: a relative position determiner determining a relative position of the normal person and the circulatory patient with respect to the user according to the calculated distance and direction; and an arrangement result outputter arranging the user, the normal person, and the circulatory patient according to the determined relative position and outputting a result of the arrangement.

The user data receiver may further receive information about a group number, and the result outputter may detect a normal person and a circulatory patient in a group to which the user belongs, among at least two groups classified according to the received group number and the result of the comparison and output the detected normal person and circulatory patient together with the user.

According to another aspect of the present disclosure, a computer-readable recording medium has recorded thereon a program for performing a method of determining circulatory disease potential.

### MODE OF DISCLOSURE

The present disclosure may include various embodiments and modifications, and certain embodiments are illustrated in the drawings and will be described below in detail. The effects and features of the present disclosure and the accomplishing methods thereof will become apparent from the following description of the embodiments taken in conjunction with the accompanying drawings. However, the present disclosure is not limited to the embodiments described below, and may be embodied in various modes.

Hereinafter, embodiments of the present disclosure will be described in detail with reference to the accompanying drawings. In the following description, like reference numerals will denote like elements, and redundant descriptions thereof will be omitted.

It will be understood that although the terms "first", "second", etc. may be used herein to describe various components, these components should not be limited by these terms. These terms are only used to distinguish one component from another component.

As used herein, the singular forms "a", "an", and "the" are intended to include the plural forms as well, unless the context clearly indicates otherwise.

It will be understood that the terms "comprise", "include", and "have" used herein specify the presence of stated features or components, but do not preclude the presence or addition of one or more other features or components.

When a certain embodiment may be implemented differently, a particular process order may be performed differently from the described order. For example, two consecutively described processes may be performed substantially at the same time or performed in an order opposite to the described order.

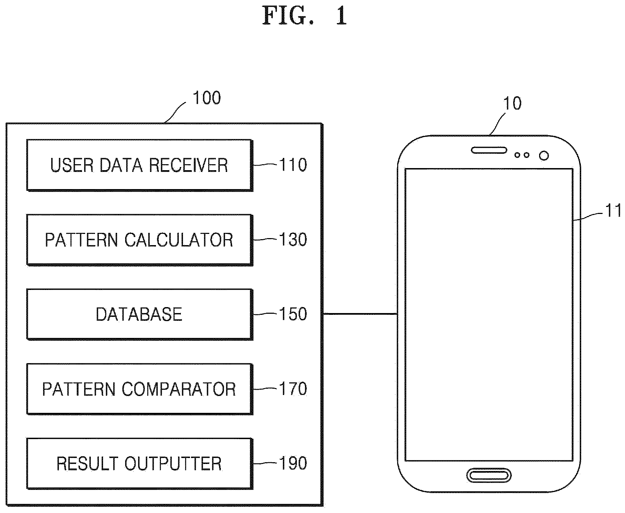

FIG. 1 is a block diagram illustrating an example of a circulatory disease potential determining apparatus included in a user terminal.

A user terminal 10 illustrated together with a circulatory disease potential determining apparatus 100 according to an embodiment of the present disclosure may include a display unit for displaying a screen 11 and an input device for receiving an input of data from the user. The input device included in the user terminal 10 may be at least one of a keyboard, a mouse, a trackball, a microphone, a mechanical button, and a touch panel.

Referring to FIG. 1, the user terminal 10 is illustrated as a smart phone. However, when the present disclosure is implemented, the user terminal 10 is not limited to a smart phone and may include any portable terminal such as a tablet personal computer (PC) or a netbook.

The circulatory disease potential determining apparatus 100 according to an embodiment of the present disclosure may correspond to at least one processor or may include at least one processor. Accordingly, the circulatory disease potential determining apparatus 100 may be driven while being included in a hardware device such as a microprocessor or a general-purpose computer system. That is, the circulatory disease potential determining apparatus 100 may be installed as hardware in the user terminal 10 or may be connected to the user terminal 10 through a wired/wireless network to control the content displayed on the screen 11 of the user terminal 10. The circulatory disease potential determining apparatus 100 illustrated in FIG. 1 illustrates only components for highlighting the features of embodiments of the present disclosure. Thus, those of ordinary skill in the art will understand that other general-purpose components other than the components illustrated in FIG. 1 may be further included according to other embodiments and the embodiment illustrated in FIG. 1.

The circulatory disease potential determining apparatus 100 according to an embodiment of the present disclosure may control the content displayed on the screen 11 of the user terminal 10. More particularly, the circulatory disease potential determining apparatus 100 may control the content displayed on the screen 11 of the user terminal 10 to be displayed differently according to the input of the user and may perform a processing operation to output information about a circulatory disease that the user is likely to catch.

Referring to FIG. 1, the circulatory disease potential determining apparatus 100 according to an embodiment of the present disclosure may include a user data receiver 110, a pattern calculator 130, a database 150, a pattern comparator 170, and a result outputter 190.

First, the user data receiver 110 may receive data about the blood pressure and blood flow of the user. Here, the data about the blood pressure may include all data such as the maximum blood pressure, the minimum blood pressure, and the difference between the maximum blood pressure and the minimum blood pressure of the user, and the data about the blood flow may be data about the flow of blood varying directly or indirectly according to the heartbeat of the user and may include various data such as a heart rate, a pulse (a pulse rate), an electrocardiogram, and a heart sound.

Particularly, the heartbeat may refer to a pulsation in which the heart itself moves, the pulse may refer to a periodic pulsation generated when blood is ejected from the heart to contact the artery, and both data may not have a great difference therebetween. Therefore, hereinafter, both the heartbeat and the pulse may be mixedly used as data representing the data about the blood flow.

As an example, the user data receiver 110 may receive user data about the maximum blood pressure, minimum blood pressure, and pulse of the user. When the user measures the user's own blood pressure and pulse through a portable blood pressure meter and a pulse meter, the blood pressure meter and the pulse meter may transmit the measured values to the user data receiver 110. In this case, the values measured by the blood pressure meter and the pulse meter may be transmitted to the user data receiver 110 not only through wired communication such as cable connection but also through a near field communication (NFC) function such as Bluetooth.

The data about the maximum blood pressure, minimum blood pressure, and pulse of the user received by the user data receiver 110 may not refer to the maximum blood pressure, the minimum blood pressure, and the pulse that are measured at a particular moment and may refer to data that are received from the blood pressure meter and the pulse meter periodically at least two times for a preset limited time.

For example, when the user suffering from a cardiovascular disease wears a wearable blood pressure and pulse measurement apparatus for 18 hours that is the average activity time per day and the measurement apparatus automatically measures the blood pressure and pulse of the user at every 30 minutes according to the period set in the measurement apparatus, the limited time may be 18 hours, the measurement period may be 30 minutes, and the maximum blood pressure, minimum blood pressure, and pulse sets measured and accumulated for one day may be 37 sets including the first measured set.

The user's maximum blood pressure, minimum blood pressure, and pulse may not be significant as each separate index. However, when these three indexes are considered together, they considerably reflect the constitutional characteristics of the user. In practice, such indexes are known as indexes that are used to discriminate a coronary artery disease, an acute myocardial infarction, an unstable angina pectoris, a stable heart disease, an old myocardial infarction, a cerebral infarction, a peripheral vascular disease, and the like, together with the test results obtained by doctors through various measurement apparatuses in hospitals.

The pattern calculator 130 may calculate a three-dimensional (3D) user pattern for the user based on the data received by the user data receiver 110. The data received by the user data receiver 110 may be three types of data such as the maximum blood pressure, minimum blood pressure, and pulse of the user and may not refer to the maximum blood pressure, minimum blood pressure, and pulse only at a particular moment, as described above.

The 3D user pattern calculated by the pattern calculator 130 may reflect the characteristics of the maximum blood pressure, minimum blood pressure, and pulse of the user as they are and may be unique to the maximum blood pressure, minimum blood pressure, and pulse according to the constitution of the user. The maximum blood pressure, maximum blood pressure, and pulse periodically input with time may be represented as a time-series function and may have unique characteristics for each user. Therefore, even when a particular transformation is performed, the unique characteristics of the user may be maintained as they are.

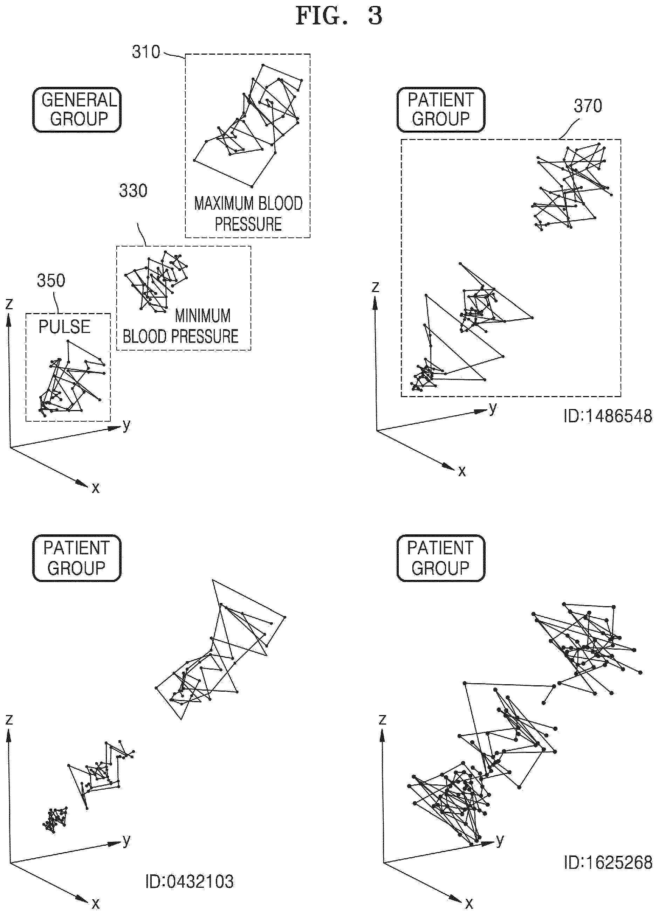

The pattern calculator 130 may apply various transformations to data received from the user data receiver 110, detect a coefficient representing the data, and project the same on 3D coordinates based on the coefficient to calculate a 3D pattern for the user. This will be described in more detail with reference to FIG. 3.

The database 150 may store a comparison target pattern that is calculated based on the maximum blood pressures, minimum blood pressures, and pulses or normal persons and circulatory patients. Here, the normal person may refer to a person that does not suffer from a circulatory disease, and the circulatory patient may refer to a person that is judged to have a circulatory disease by a doctor. The comparison target pattern stored in the database 150 may be the result of transforming data about the maximum blood pressures, minimum blood pressures, and pulses of the normal person and the circulatory patient by various transformation methods applied to the user data by the pattern calculator 130 and may refer to a pattern that is compared with the user pattern by the pattern comparator 170 described below.

Like the user data, the maximum blood pressures, minimum blood pressures, and pulses of the normal person and the circulatory patients for calculating the comparison target pattern may also be a series of values that are measured periodically for a preset limited time.

The pattern comparator 170 may compare the comparison target pattern of the normal person and the circulatory patient stored in the database 150, with the user pattern. The user pattern calculated by the pattern calculator 130 may be a 3D pattern reflecting all of the temporal change characteristics and unique characteristics of the maximum blood pressure, minimum blood pressure, and pulse of the user, and the comparison target pattern may also be a pattern having a shape in a 3D space. The pattern comparator 170 may compare and determine the similarity between the user pattern and the comparison target pattern and transmit the comparison result to the result outputter 190.

The result outputter 190 may output, based on the comparison result of the user pattern and the comparison target pattern by the pattern comparator 170, the circulatory disease potential visualizing to which of the normal person and the circulatory patient stored in the database 150 the user is closer. Here, the circulatory disease potential may be the result of quantifying and visualizing how much a circulatory disease is likely to occur in the user. According to the present disclosure, the user may input his/her own maximum blood pressure, minimum blood pressure, and pulse as the user data. Thus, even when the user has already caught or has not caught a circulatory disease, the user may easily identify how much the user is likely to catch a circulatory disease through the result of catching a circulatory disease by a person having a similar constitution to the user.

FIG. 1 illustrates an embodiment of the circulatory disease potential determining apparatus 100 according to the present disclosure. According to an embodiment, the circulatory disease potential determining apparatus 100 according to the present disclosure may be connected to a multifunctional intelligent complex terminal such as a mobile phone by wire or wireless, may be included as hardware or software in a multifunctional intelligent complex terminal, or may be implemented as an apparatus having an integrated configuration for finally outputting the circulatory disease potential to the user while including a measurement apparatus such as a blood pressure meter or a pulse meter even without a multifunctional intelligent complex terminal.

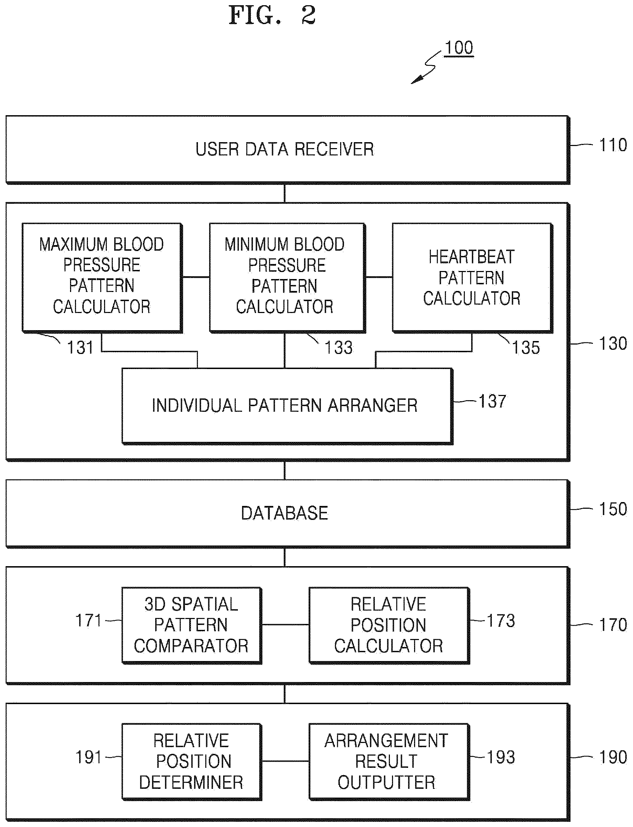

FIG. 2 is a block diagram illustrating another example of a circulatory disease potential determining apparatus according to the present disclosure.

Referring to FIG. 2, a circulatory disease potential determining apparatus 100 according to the present disclosure may include a user data receiver 110, a pattern calculator 130, a database 150, a pattern comparator 170, and a result outputter 190. In order to avoid the confusion between components, the same reference numerals will be used for the same components as illustrated in FIG. 1.

First, the user data receiver 110 may receive user data about the maximum blood pressure, minimum blood pressure, and pulse of the user. Here, the user data may be data of the user periodically measured for a certain limited time.

In an alternative embodiment, the limited time and the data measurement period may vary depending on the user's input. More particularly, when the user inputs a value for the limited time and the measurement period through an input device included in the user terminal 10, the user data receiver 110 may receive the measurement result of a blood pressure meter and a pulse meter and consider the same as the user data.

For example, when it is set such that the user inputs "24 hours" as the limited time and the blood pressure and pulse meter measures the user's blood pressure and pulse at every 30 minutes for the activity time from 9:00 AM to the next day midnight and measures the user's blood pressure and pulse at every hour for the sleep time, the user data receiver 110 may receive, as the user data, the data measured at least two times for the input time according to the input period. As another example, when the user inputs "120 hours (5 days)" as the limited time and sets "1 hour" as the measurement period of the blood pressure and pulse meter, the user data receiver 110 may receive, as the user data, the data measured at least two times for the input time according to the input period.

When the user inputs particular values according to his/her own body rhythm and activity period, since the user data receiver 110 may receive different user data according to a different limited time and measurement period for each user, the user data receiver 110 may receive user data reflecting the unique characteristics of the user.

Subsequently, the pattern calculator 130 may calculate a 3D user pattern for the user based on the user data. The user data may include data about the user's maximum blood pressure, minimum blood pressure, and pulse. However, since the blood pressure and the pulse may be different from each other in terms of the display unit and the value itself alone may have a particular meaning only under the clinical judgment of a doctor, the pattern calculator 130 may apply various transformations to the user data to calculate a 3D user pattern in order to represent the unique characteristics of the user as they are and simultaneously represent the association with the user data of the next period periodically received with time.

As an example, the pattern calculator 130 may include a 3D coordinate dictionary (not illustrated) for coordinates respectively corresponding to the values of the maximum blood pressure, minimum blood pressure, and pulse. For example, when the maximum blood pressure of the user at about 2:00 PM is 130 mmHg, since the corresponding 3D coordinate value (x-axis coordinate, y-axis coordinate, z-axis coordinate) is stored in the 3D coordinate dictionary (not illustrated), the pattern calculator 130 may calculate a 3D coordinate value as a portion of the 3D user pattern with reference to the stored 3D coordinate value.

As another example, the pattern calculator 130 may model each of the maximum blood pressure, minimum blood pressure, and pulse input with time to the user data receiver 110 as a time-series function and then generate a 3D vector in a 3D space according to a time delay embedding process based on the time-series function and the coordinates for the time-series function to calculate the user pattern. Since the time delay embedding process of the present embodiment is based on the generally-known technology (e.g., the published document described in the Background Art section of the specification and Korean Patent Application Publication No. 2016-0094317), redundant descriptions thereof will be omitted for conciseness.

According to an embodiment, the pattern calculator 130 may include a maximum blood pressure pattern calculator 131, a minimum blood pressure pattern calculator 133, a heartbeat pattern calculator 135, and an individual pattern arranger 137.

The maximum blood pressure pattern calculator 131 may calculate the user maximum blood pressure pattern based on the user data about the maximum blood pressure of the user. After the user data receiver 110 periodically receives the user data with the lapse of time, the maximum blood pressure pattern calculator 131 may calculate the user maximum blood pressure pattern by extracting only the maximum blood pressure from the user data.

The minimum blood pressure pattern calculator 133 may calculate the user minimum blood pressure pattern based on the user data about the minimum blood pressure of the user. After the user data receiver 110 periodically receives the user data with the lapse of time, the minimum blood pressure pattern calculator 133 may calculate the user minimum blood pressure pattern by extracting only the minimum blood pressure from the user data.

The heartbeat pattern calculator 135 may calculate the user pulse pattern based on the user data about the heartbeat of the user.

After the user data receiver 110 periodically receives the user data with the lapse of time, the heartbeat pattern calculator 135 may calculate the user pulse pattern by extracting only the pulse from the user data. The user maximum blood pressure pattern, the user minimum blood pressure pattern, and the user pulse pattern may all be represented as a vector in a 3D space, and a process of representing these three patterns may be based on the 3D coordinate dictionary or the time delay embedding process described above.

The individual pattern arranger 137 may arrange the three patterns calculated by the maximum blood pressure pattern calculator 131, the minimum blood pressure pattern calculator 133, and the heartbeat pattern calculator 135 together in a single 3D space. The user maximum blood pressure pattern, the user minimum blood pressure pattern, and the user pulse pattern arranged together in the single 3D space may reflect the unique constitutional characteristics of the user as before the processing of the patterns.

FIG. 3 is a diagram illustrating an example of a 3D user pattern calculated by a pattern calculator.

Referring to FIG. 3, the 3D user pattern calculated by the pattern calculator may be a pattern in which a user maximum blood pressure pattern 310, a user minimum blood pressure pattern 330, and a user pulse pattern 350 are arranged in a single 3D space, and a comparison target pattern 370 compared with the user pattern by the pattern comparator 170 described below may be next thereto.

The individual pattern arranger 137 may arrange the user maximum blood pressure pattern 310, the user minimum blood pressure pattern 330, and the user pulse pattern 350 in a single 3D space according to the respective 3D coordinate values thereof. FIG. 3 illustrates that the user maximum blood pressure pattern 310, the user minimum blood pressure pattern 330, and the user pulse pattern 350 are arranged in a 3D space of the x axis, y axis, and z axis.

Referring to FIG. 3, when the 3D coordinate values of the user's maximum blood pressure, minimum blood pressure, and pulse at a particular time are determined by the 3D coordinate dictionary described above, the respective patterns may be the results of vector-connecting the determined 3D coordinate values with the lapse of time. For example, when the user's maximum blood pressure at about 2:00 PM is 130 mmHg and the user's maximum blood pressure at about 2:10 PM is 120 mmHg, the user's maximum blood pressure pattern may be obtained by connecting, by a vector, the corresponding 3D coordinate values for the case where the maximum blood pressure is 120 mmHg at 2:10 PM from the corresponding 3D coordinate values (x-axis coordinate, y-axis coordinate, z-axis coordinate) for the case where the maximum blood pressure is 130 mmHg at 2:00 PM.

Since the user maximum blood pressure pattern 310, the user minimum blood pressure pattern 330, and the user pulse pattern 350 are significantly different in terms of the values before being patterned, when they are respectively arranged as a 3D vector form in a single 3D space after being patterned, they may be generally spaced apart from each other by a certain distance. However, when the user's health condition changes suddenly or the user suffers from a circulatory disease, the boundary division of the maximum blood pressure pattern, the minimum blood pressure pattern, and the pulse pattern may be difficult as in various comparison target patterns illustrated in FIG. 3 and such an overall shape itself may be a factor reflecting the constitutional characteristics of the user.

Hereinafter, a description given with reference to FIG. 2 will be again given below.

The database 150 may store a comparison target pattern that is calculated based on the maximum blood pressures, minimum blood pressures, and pulses of normal persons and circulatory patients. Here, a normal person may refer to a person that does not suffer from a circulatory disease, and a circulatory patient may refer to a person that is judged to have a circulatory disease by a doctor. The comparison target pattern stored in the database 150 may be the result of transforming data about the maximum blood pressures, minimum blood pressures, and pulses of the normal person and the circulatory patient by various transformation methods applied to the user data by the pattern calculator 130 and may be a pattern that is compared with the user pattern by the pattern comparator 170 described below.

The maximum blood pressures, minimum blood pressures, and pulses of the normal person and circulatory patient for calculating the comparison target pattern may be a series of values measured periodically for a certain limited time like the user data, the interval and amount of the user data being received by the user data receiver 110 may vary due to the change of the limited time and the period by the user's input according to the alternative embodiment described above, and thus the measured data may be stored in the database at short time intervals.

The pattern comparator 170 may compare the comparison target pattern stored in the database 150 with the user pattern and may include a 3D spatial pattern comparator 171 and a relative position calculator 173.

The 3D spatial pattern comparator 171 may compare the user pattern to the comparison target pattern based on the position on a 3D space. First, the 3D spatial pattern comparator 171 may compare and determine how much the comparison target pattern and the user pattern existing in the 3D space are similar to each other in terms of their respective morphological features. When the morphological features of both patterns compared with each other are similar to each other, the 3D spatial pattern comparator 171 may compare and determine how much their 3D coordinate values are similar to each other when they are located in the same 3D space. In this process, the position of the center point of each pattern may be considered, only the maximum blood pressure pattern of the user pattern and the maximum blood pressure pattern of the comparison target pattern may be compared with each other, and the patterns calculated by different item values, for example, the minimum blood pressure pattern and the pulse pattern, may not be compared with each other in terms of similarity.

The relative position calculator 173 may receive the comparison result of the 3D spatial pattern comparator 171 and calculate the distance and direction between the user pattern and the comparison target pattern based on the comparison result. As an example, when the user pattern and the comparison target pattern are substantially similar to each other in the 3D spatial pattern comparator 171, the distance calculated by the relative position calculator 173 may be a small value, and when the user pattern and the comparison target pattern are not similar to each other, the direction calculated by the relative position calculator 173 may be the opposite.

The relative position calculator 173 may compare the comparison target pattern for all objects (normal persons and circulatory patients) stored in the database 150 with the user pattern one by one to calculate a relative position and transmit the relative position to a relative position determiner 191.

The result outputter 190 may output, based on the comparison result of the pattern comparator 170, the circulatory disease potential visualizing to which of the normal person and the circulatory patient the user is closer and may include a relative position determiner 191 and an arrangement result outputter 193.

The relative position determiner 191 may receive the value of the distance and direction between the user pattern and the comparison target pattern from the relative position calculator 173 and determine the relative position of the normal person and the circulatory patient with respect to the user according to the received distance and direction.

The information received from the relative position calculator 173 by the relative position determiner 191 may correspond to the product of the number of comparison target patterns and the result of calculating the relative position by comparing one comparison target pattern with the user pattern, and the relative position determiner 191 may determine the relative position of the normal person and the circulatory patient with respect to the user according to the relative position calculated by the relative position calculator 173. Therefore, in this process, the relative position between the normal person and the circulatory patient stored in the database may also need to be determined, the relative position therebetween may be information prestored in the database 150, and the relative position determiner 191 may receive and use the information from the database 150.

Since the value calculated by the relative position calculator 173 may be the distance and direction between the user pattern and the comparison target pattern, the relative position determined by the relative position determiner 191 may visualize how similar constitution the normal person or the circulatory patient has with respect to the user in terms of the maximum blood pressure, minimum blood pressure, and pulse. According to an embodiment, the relative position calculator 173 may also perform the function performed by the relative position determiner 191, and the relative position determiner 191 may be omitted from the result outputter 190.

Subsequently, when the relative position determiner 191 determines the relative position of the normal person and the circulatory patient in the 3D space with respect to the user based on the distance and direction between the user pattern and the comparison target pattern, the arrangement result outputter 193 may arrange the normal person and the circulatory patient according to the determined position and visualize and output the arrangement result.

Hereinafter, the result output by the arrangement result outputter 193 will be referred to as a similarity map. The similarity map may be a visual mark that directly indicates, in terms of the maximum blood pressure, minimum blood pressure, and pulse among the comparison target persons stored in the database, which person has a similar constitution to the user and how many persons among the persons having a similar constitution to the user are circulatory patients.

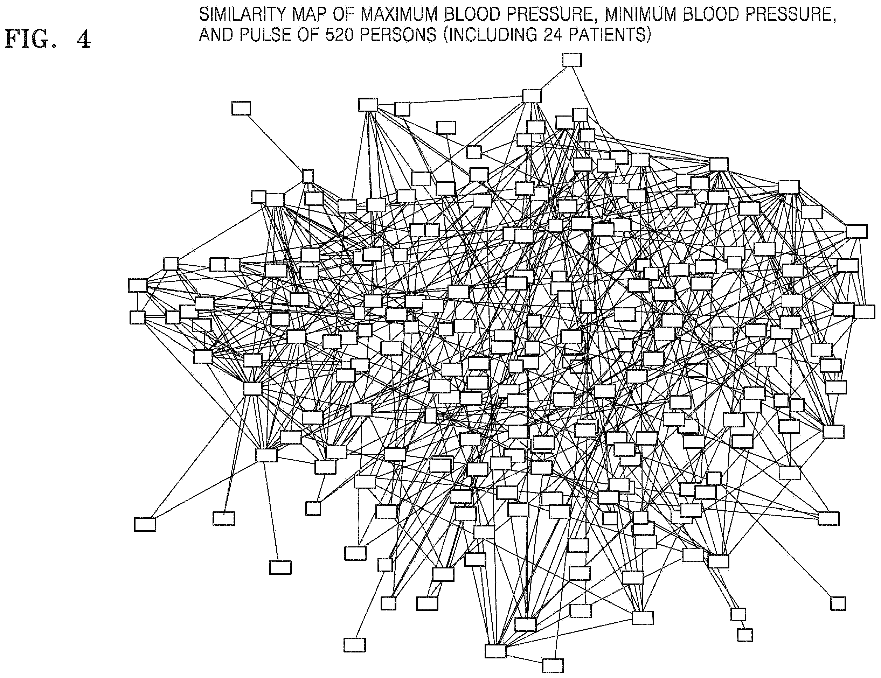

FIG. 4 is a diagram illustrating an example of a similarity map output by a result outputter.

Referring to FIG. 4, it may be seen that the relative positions of 520 comparison target persons are determined with respect to the user and 24 persons among the 520 persons are definitely diagnosed as circulatory patients. When the user and 520 comparison target persons are regarded as respective nodes, FIG. 4 may be interpreted such that different nodes have more similar constitutions as the distance therebetween decreases, and the second node and the third node have dissimilar constitutions when the second node and the third node are located at the same distance from the first node and are opposite to each other with respect to the first node.

In an alternative embodiment, the user data receiver 110 may further receive information about the number of groups (i.e., a group number) from the user. In this case, the result outputter 190 may output, together with the user, the result of the comparison of the user pattern and the comparison target pattern by the pattern comparator 170 and the result of the detection of the normal person and the circulatory patient in the group to which the user belongs among at least two groups classified according to the number of groups (i.e., the group number) received by the user data receiver 110.

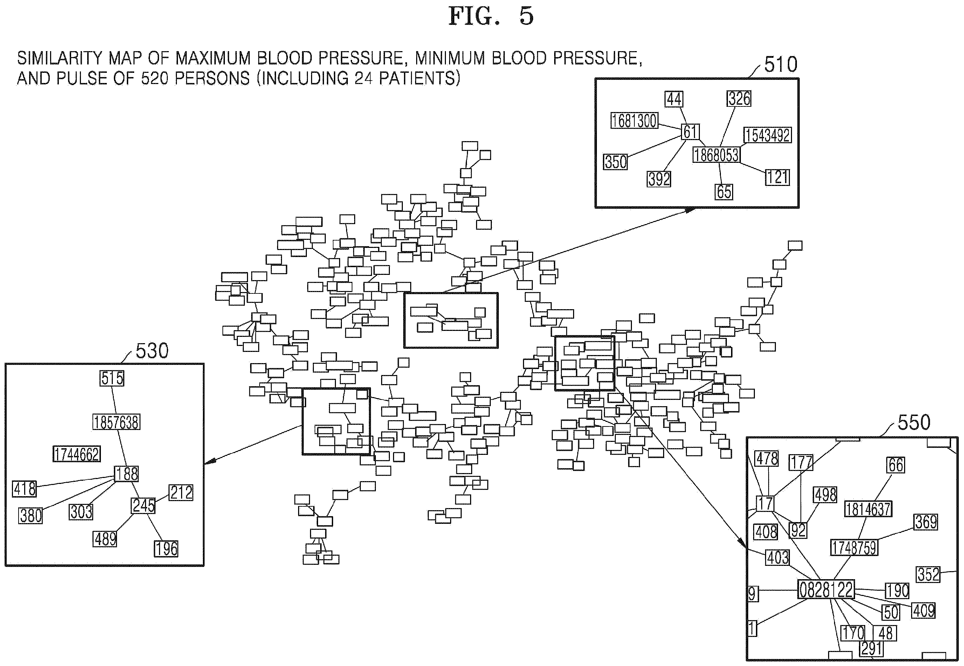

FIG. 5 is a diagram illustrating an example of a similarity map output when a user data receiver receives information about the number of groups.

Reference will be made to FIG. 4 in order to facilitate the description given with reference to FIG. 5.

Referring to FIG. 5, it may be seen that the similarity map illustrated in FIG. 4 is divided into several groups. A plurality of groups constituting the similarity map may be classified according to the number of groups input by the user. In the present disclosure, when information about the number of groups is received, a method of classifying the similarity map according to the number of groups is limited to a particular method and various methods including a support vector machine (SVM) may be applied thereto.

About 10 nodes may belong to one group as in a first group 510 and a second group 530 or a large number of nodes may belong to one group as in a third group 550 and a person corresponding to a node belonging to the third group 550 may be regarded as a person having the most general constitution.

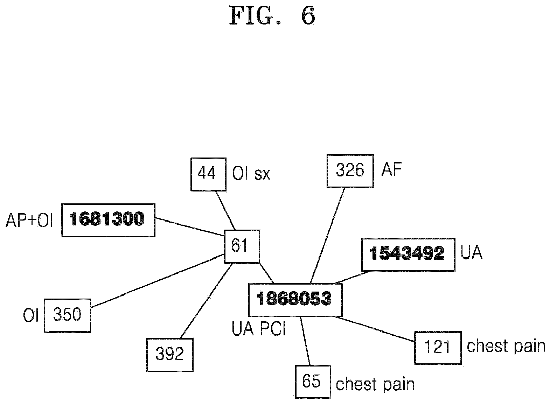

FIG. 6 is a diagram illustrating in detail the first group to which the user belongs.

Referring to FIG. 6, when a total of 10 persons belong to the first group 510 and a person denoted by "61" is regarded as the user, 5 persons directly connected to the user (i.e., persons corresponding to nodes respectively denoted by "44", "350", "392", "1868053", and "1681300") may be regarded as persons having similar constitutions to the user in the first group 510 and a person corresponding to the node "1868053" located at the shortest distance from "61" may be regarded as a person having the most similar constitution to the user because the distance between nodes is the similarity therebetween.

Assuming that a circulatory patient is represented by a 7-digit node, FIG. 6 may be interpreted such that the similarity map is classified into several groups according to the number of groups input by the user and a person having the most similar constitution to the user among 9 persons of the group to which the user belongs among the several groups may be regarded as suffering from a circulatory disease called "Unstable Angina pectoris (UA)". In addition, the disease suffered by the persons belonging to the same group may be marked as a reference next to the node, and the disease name marked next to the node may be based on the information stored in the database 150.

According to the above configuration, as the number of groups input by the user increases, the number of persons belonging to the group to which the user belongs may increase, and when the user has an unusual constitution, an extremely small number of persons may belong to the group to which the user belongs, regardless of the number of groups received by the user data receiver 110.

According to the alternative embodiment, by not inputting information about the number of groups, the user may visually identify the similarity map for comparing the constitutions of all the persons stored in the database with the user's constitution and intuitively detect the circulatory disease potential (potential risk) of the user, and by inputting information about the number of groups, the user may specify a group including only some persons determined to have similar constitutions to the user among the persons stored in the database and identify information about the persons belonging to the group, to rapidly cope with a circulatory disease that the user is likely to catch.

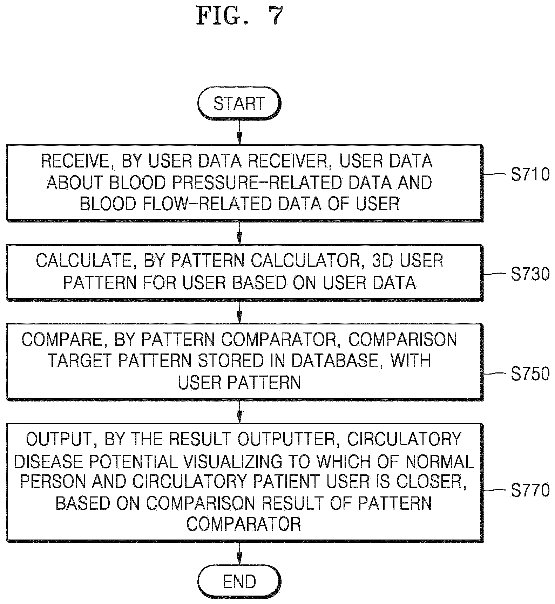

FIG. 7 is a flowchart illustrating an example of a circulatory disease potential determining method according to the present disclosure.

Since a method according to FIG. 7 may be implemented by the circulatory disease potential determining apparatus illustrated in FIGS. 1 and 2, the method will be described with reference to FIGS. 1 and 2 and redundant descriptions previously given above with reference to FIGS. 1 and 2 will be omitted for conciseness.

First, the user data receiver may receive user data about the maximum blood pressure, minimum blood pressure, and pulse of the user (operation S710).

In an alternative embodiment of operation S710, the user data may be data measured periodically at least two times for a time input by the user.

Subsequently, the pattern calculator may calculate a 3D user pattern for the user based on the user data received by the user data receiver (operation S730).

In an alternative embodiment of operation S730, the pattern calculator may calculate the user maximum blood pressure pattern, the user minimum blood pressure pattern, and the user pulse pattern based on the user data and arrange the calculated patterns together in a single 3D space, to calculate the 3D user pattern.

The pattern comparator may compare the comparison target pattern calculated based on the maximum blood pressures, minimum blood pressures, and pulses of the normal person and the circulatory patient stored in the database, with the user pattern (operation S750).

In an alternative embodiment of operation S750, the pattern comparator may compare the user pattern to the comparison target pattern based on the position in a 3D space and calculate the relative position as a result of the comparison such that the result outputter may output a similarity map for the user. The similarity map may be for the user to visually determine the circulatory disease potential, which has been described above with reference to FIGS. 5 and 6.

The result outputter may output the circulatory disease potential visualizing to which of the normal person and the circulatory patient the user is closer, based on the comparison result of the user pattern and the comparison target pattern in operation S750 (operation S770).

The embodiments of the present disclosure described above may be implemented in the form of computer programs that may be executed through various components on a computer, and the computer programs may be recorded in computer-readable recording mediums. Examples of the computer-readable recording mediums may include magnetic recording mediums such as hard disks, floppy disks, and magnetic tapes, optical recording mediums such as CD-ROMs and DVDs, magneto-optical recording mediums such as floptical disks, and hardware devices such as ROMs, RAMs and flash memories that are especially configured to store and execute program commands.

The computer programs may be those that are especially designed and configured for the present disclosure, or may be those that are known and available to computer programmers skilled in the art. Examples of the computer programs may include machine language code that may be generated by a compiler, and high-level language code that may be executed by a computer by using an interpreter.

Particular implementations described herein are merely embodiments, and do not limit the scope of the present disclosure in any way. For the sake of conciseness, descriptions of related art electronic configurations, control systems, software, and other functional aspects of the systems may be omitted. Also, the connection lines or connection members between various components illustrated in the drawings represent examples of functional connections and/or physical or logical connections between the various components, and various alternative or additional functional connections, physical connections, or logical connections may be present in practical apparatuses. Also, no element may be essential to the practice of the present disclosure unless the element is particularly described as "essential" or "critical".

The use of the terms "a", "an", and "the" and similar referents in the context of the specification (especially in the context of the following claims) may be construed to cover both the singular and the plural. Also, recitation of a range of values herein are merely intended to serve as a shorthand method of referring individually to each separate value falling within the range, unless otherwise indicated herein, and each separate value is incorporated into the specification as if it was individually recited herein. Also, the operations of the method described herein may be performed in any suitable order unless otherwise indicated herein or otherwise clearly contradicted by the context. The scope of the present disclosure is not limited to the above-described operation order. All examples or example terms (e.g., "such as") provided herein are merely used to describe the present disclosure in detail, and the scope of the present disclosure is not limited by the examples or example terms unless otherwise claimed. Also, those of ordinary skill in the art will readily understand that various modifications and combinations may be made according to design conditions and factors without departing from the spirit and scope of the present disclosure as defined by the following claims.

### INDUSTRIAL APPLICABILITY

The present disclosure may be used to develop an apparatus for self-diagnosing a health condition of a user.

## Claims

1. A method of determining circulatory disease potential of a user, the method comprising:
a user data receiving operation of receiving user data including data about a series of maximum blood pressures and data about a series of minimum blood pressures varying with time, which are measured and transmitted from a blood pressure meter periodically a plurality of times for a limited time input, and data about a series of pulses varying with time, which are measured and transmitted from a pulse meter periodically a plurality of times for the limited time;
a pattern calculating operation of calculating a three-dimensional (3D) user pattern for the user based on the received user data;
a pattern comparing operation of comparing a comparison target pattern calculated for each of a maximum blood pressure, a minimum blood pressure, and a pulse based on data about maximum blood pressures, minimum blood pressures, and blood flows of a normal person and a circulatory patient stored in a database, with the calculated 3D user pattern; and
a result output operation of outputting, based on a result of the comparison, circulatory disease potential visualizing to which of the normal person and the circulatory patient stored in the database the user is closer,
wherein the pattern calculating operation calculates a 3D vector pattern for each of the maximum blood pressure, the minimum blood pressure, and the pulse based on the fact that the data about the maximum blood pressure, the minimum blood pressure, and the pulse included in the user data varies with time.

2. The method of claim 1, wherein the pattern calculating operation comprises an individual pattern arranging operation of arranging the calculated 3D vector pattern for the maximum blood pressure, the calculated 3D vector pattern for the minimum blood pressure, and the calculated 3D vector pattern for the pulse together in a single 3D space, and
the pattern comparing operation arranges, in the single 3D space, the comparison target pattern calculated for each of the maximum blood pressure, the minimum blood pressure, and the pulse and compares, in the single 3D space, the calculated 3D vector pattern for the maximum blood pressure, the calculated 3D vector pattern for the minimum blood pressure, and the calculated 3D vector pattern for the pulse with the comparison target pattern calculated for each of the maximum blood pressure, the minimum blood pressure, and the pulse, respectively.

3. The method of claim 1, wherein the pattern comparing operation comprises:
a 3D spatial pattern comparing operation of comparing the comparison target pattern with the calculated 3D user pattern based on a position in a 3D space; and
a relative position calculating operation of calculating a distance and direction of the calculated 3D user pattern and the comparison target pattern based on a result of the comparison in the 3D spatial pattern comparing operation, and
the result output operation comprises:
a relative position determining operation of determining a relative position of the normal person and the circulatory patient with respect to the user according to the calculated distance and direction; and
an arrangement result output operation of arranging the user, the normal person, and the circulatory patient according to the determined relative position and outputting a result of the arrangement.

4. The method of claim 1, further comprising a group number information receiving operation of receiving information about a group number,
wherein the result output operation classifies the normal person, the circulatory patient, and the user into at least two groups according to the received group number and a similarity between the 3D user pattern and the comparison target pattern based on the result of the comparison, detects a normal person and a circulatory patient belonging to a group to which the user belongs, among the at least two groups, and outputs the detected normal person and circulatory patient together with the user.

5. A non-transitory computer-readable recording medium having recorded thereon a program for performing the method of claim 1.

6. An apparatus for determining circulatory disease potential of a user, the apparatus comprising:
a database storing a comparison target pattern calculated based on data about blood pressures and blood flows of a normal person and a circulatory patient;
a user data receiver receiving user data including data about a series of maximum blood pressures and data about a series of minimum blood pressures varying with time, which are measured and transmitted from a blood pressure meter periodically a plurality of times for a limited time input, and data about a series of pulses varying with time, which are measured and transmitted from a pulse meter periodically a plurality of times for the limited time;
a pattern calculator calculating a three-dimensional (3D) user pattern for the user based on the received user data;
a pattern comparator comparing a comparison target pattern calculated for each of a maximum blood pressure, a minimum blood pressure, and a pulse based on data about maximum blood pressures, minimum blood pressures, and blood flows of a normal person and a circulatory patient stored in the database, with the calculated 3D user pattern; and
a result outputter outputting, based on a result of the comparison, circulatory disease potential visualizing to which of the normal person and the circulatory patient stored in the database the user is closer,
wherein the pattern calculator calculates a 3D vector pattern for each of the maximum blood pressure, the minimum blood pressure, and the pulse based on the fact that the data about the maximum blood pressure, the minimum blood pressure, and the pulse included in the user data varies with time.

7. The apparatus of claim 6, wherein the pattern calculator comprises an individual pattern arranger arranging the calculated 3D vector pattern for the maximum blood pressure, the calculated 3D vector pattern for the minimum blood pressure, and the calculated 3D vector pattern for the pulse together in a single 3D space, and
the pattern comparator arranges, in the single 3D space, the comparison target pattern calculated for each of the maximum blood pressure, the minimum blood pressure, and the pulse and compares, in the single 3D space, the calculated 3D vector pattern for the maximum blood pressure, the calculated 3D vector pattern for the minimum blood pressure, and the calculated 3D vector pattern for the pulse with the comparison target pattern calculated for each of the maximum blood pressure, the minimum blood pressure, and the pulse, respectively.

8. The apparatus of claim 6, wherein the pattern comparator comprises:
a 3D spatial pattern comparator comparing the comparison target pattern with the calculated 3D user pattern based on a position in a 3D space; and
a relative position calculator calculating a distance and direction of the calculated 3D user pattern and the comparison target pattern based on a result of the comparison by the 3D spatial pattern comparator, and
the result outputter comprises:
a relative position determiner determining a relative position of the normal person and the circulatory patient with respect to the user according to the calculated distance and direction; and
an arrangement result outputter arranging the user, the normal person, and the circulatory patient according to the determined relative position and outputting a result of the arrangement.

9. The apparatus of claim 6, wherein the user data receiver further receives information about a group number, and
the result outputter classifies the normal person, the circulatory patient, and the user into at least two groups according to the received group number and a similarity between the 3D user pattern and the comparison target pattern based on the result of the comparison, detects a normal person and a circulatory patient belonging to a group, to which the user belongs, among the at least two groups, and outputs the detected normal person and circulatory patient together with the user.
